# FASCICULE DE BREVET EUROPEEN

(11) **EP 3 253 664 B1**
(45) Date de publication et mention de la délivrance du brevet: **31.10.2018**
(21) Numéro de dépôt: 16703146.7
(22) Date de dépôt: 05.02.2016
(51) Int. Cl.: B65D 5/68, A61L 2/20, A61L 2/26, B65D 5/22, B65D 5/42

(54) **EMBALLAGE POUR PRODUITS STERILES**
VERPACKUNG FÜR STERILE PRODUKTE
PACKAGING FOR STERILE PRODUCTS

(30) Priorité: 05.02.2015 FR 1550917
(43) Date de publication de la demande: 13.12.2017
(73) Titulaire: DS SMITH PLASTICS FRANCE, 68240 Kaysersberg (FR)
(72) Inventeur: ANTOINE, Jacques, 68140 Stosswihr (FR); BAUMANN, Daniel, 68000 Colmar (FR); FRENEAT, Jérémy, 68170 Rixheim (FR); MAIRE, Christian, 68370 Orbey (FR); MIRANDA, Sébastien, 68740 Rumersheim le Haut (FR)
(74) Mandataire: Gevers & Orès
(86) Numéro de dépôt international: PCT/EP2016/052545
(87) Numéro de publication internationale: WO 2016/124759

(56) Documents cités:
- WO-A2-2006/095097
- FR-A1- 2 283 056
- US-A- 5 839 651

## Description

### Domaine de l'invention

La présente invention concerne une barquette avec coiffe, les deux étant réalisées chacune par le pliage et la mise en volume d'une plaque en matériau semi rigide plastique, la barquette avec coiffe étant destinée à recevoir des produits stériles.

### Exposé de l'état de la technique

Pour l'emballage et l'expédition de produits stériles, tels que des instruments ou articles médicaux ou chirurgicaux, par exemple des boîtes ou des sachets contenant des seringues, il est connu d'utiliser des caisses en matière plastique alvéolaire que l'on traite ensemble dans une enceinte de stérilisation avec les produits qu'elles contiennent. Une méthode connue de traitement de stérilisation consiste à remplir la caisse avec le produit concerné venu de fabrication et non encore stérile, puis à soumettre l'ensemble à un traitement de stérilisation par un gaz approprié, tel que l'EtO (oxyde d'éthylène) dans une enceinte hermétique. Après un temps d'exposition déterminé, jusqu'à 75 heures, le gaz est évacué de l'enceinte et remplacé par de l'air avant que l'on sorte le produit de l'enceinte. Le produit traité est ainsi expédié dans sa caisse sans autre manipulation avant son arrivée à destination.

Le gaz utilisé pour le traitement de stérilisation étant par nature très toxique puisqu'il doit supprimer tout germe, on s'assure que des traces de gaz ne restent pas emprisonnées à l'intérieur de l'emballage. Les procédures de traitement mises en oeuvre sont élaborées pour assurer une sécurité totale à l'égard des opérateurs et agents chargés de manipuler les caisses, notamment en sortie de l'enceinte de traitement.

On emploie volontiers, pour confectionner les caisses d'emballage, des plaques de matériau plastique alvéolaire. Ce matériau est à la fois léger et résistant. Il se compare au carton ondulé en termes de performances mécaniques, et présente l'avantage supplémentaire d'être durable et plus résistant à la contamination que ce dernier.

On désigne par l'expression « plaques en matériau plastique alvéolaire » des plaques constituées d'au moins deux feuilles planes parallèles maintenues à distance l'une de l'autre par des cloisons parallèles entre elles. Les feuilles et les cloisons ménagent entre elles des canaux longitudinaux que l'on désigne alvéoles dans le domaine. Ces plaques sont principalement obtenues par extrusion d'une matière plastique choisie en fonction de l'application visée pour le matériau, telle qu'une polyoléfine, à travers une filière rectiligne de profil approprié. Immédiatement en aval de la tête d'extrusion, la matière, encore à l'état plastique, passe entre des plaques de calibrage pour figer la forme de la plaque.

Ce type de plaque est maintenant d'usage courant et est susceptible de se substituer au carton ondulé pour certaines applications comme celle rapportée ci-dessus. La caisse courante est la caisse américaine. Elle comprend une ceinture à quatre panneaux prolongés chacun de part et d'autre par des rabats que l'on replie à angle droit pour former le fond et le haut ou couvercle de la caisse.

La présente demanderesse a développé une caisse utilisée pour le traitement de stérilisation, protégée par brevet EP 1858760, dans laquelle on perfore les feuilles de couverture constituant la plaque alvéolaire d'une multitude de trous pour assurer une circulation du gaz à travers celle-ci pendant la phase de traitement dans l'enceinte. L'objectif était d'éviter que le gaz s'accumule et forme des poches résiduelles en fin de traitement lors de la phase d'élimination du gaz de stérilisation avec en particulier une circulation des gaz améliorée à travers la caisse quand les rabats sont pliés à angle droit et la caisse refermée prête à être expédiée directement après son traitement dans l'enceinte de stérilisation gazeuse.

La caisse selon l'art antérieur illustré par ce brevet est constituée de panneaux adjacents formant une ceinture et des rabats prolongeant les panneaux pour le fond et le dessus. La ceinture et les rabats sont découpés dans une plaque en matériau plastique alvéolaire comportant au moins deux feuilles de couverture maintenues à distance l'une de l'autre par une pluralité d'entretoises ménageant des alvéoles longitudinales parallèles entre elles. Au moins l'une des feuilles de couverture de la caisse est perforée pour permettre le passage d'un gaz de stérilisation au travers de ladite feuille de couverture. Les perforations permettent le passage du gaz mais sont suffisamment petites pour empêcher le passage des poussières. En outre des découpes larges traversent au moins l'autre feuille de couverture de la plaque pour assurer la communication des alvéoles avec l'extérieur.

Cette solution procure une grande sécurité quant à la circulation des gaz et surtout en ce qui concerne l'élimination après traitement. En effet les alvéoles forment des cheminées débouchant directement à l'extérieur grâce à ces découpes. Ces découpes sont suffisamment larges pour chevaucher au moins la largeur d'une alvéole. De préférence les perforations sont ménagées sur la feuille de la plaque à l'intérieur de la caisse et les découpes sur la feuille extérieure. Les découpes sont ménagées le long de lignes d'articulation des rabats sur la ceinture.

L'invention consiste à appliquer un agencement semblable à un emballage comprenant une barquette et une coiffe recouvrant la barquette. Un autre emballage permettant la circulation de gaz est connu du document US 5 839 651.

### Exposé de l'invention

On parvient à la solution conformément à l'invention avec emballage pour produits stériles comprenant une barquette et une coiffe en matériau plastique alvéolaire comportant au moins deux feuilles de couverture maintenues à distance l'une de l'autre par une pluralité d'entretoises ménageant des alvéoles longitudinales parallèles entre elles, la barquette et la coiffe comprenant un fond et des parois latérales découpés dans une plaque dudit matériau et dont au moins une desdites parois latérales est à double épaisseur et formée par pliage de deux panneaux de la plaque autour de lignes de pliage perpendiculaires à la direction desdites entretoises, emballage caractérisé par le fait que
Les feuilles de couverture intérieures sont perforées pour permettre le passage d'un gaz de stérilisation au travers de ladite feuille de couverture, les perforations permettant le passage du gaz mais empêchant le passage des poussières,
Des découpes larges traversent au moins l'autre feuille de couverture de la plaque pour assurer la communication desdites alvéoles avec l'extérieur et sont ménagées le long des lignes de pliage desdits panneaux.

L'emballage de l'invention présente les caractéristiques additionnelles suivantes prises seules ou en combinaison :
Les perforations sont ménagées sur la feuille de la plaque à l'intérieur de la caisse, et les découpes sur la feuille extérieure.
Des ouvertures sont pratiquées le long de la ligne de pliage entre la paroi double et le fond ces ouvertures ayant pour fonction de recevoir des ergots de verrouillage sur le bord libre du panneau extérieur et étant agencées pour permettre la circulation de gaz dans l'emballage depuis l'extérieur.
Des découpes sont pratiquées le long de ladite ligne de pliage de fond, les découpes et les ouvertures étant disposées en quinconce par rapport aux découpes sur la ligne de pliage des panneaux entre eux.
Une partie des alvéoles débouche sur des découpes à leurs deux extrémités.
La barquette comprend une seule paroi latérale à double panneaux.
Les parois adjacentes à ladite paroi latérale double comprennent des volets disposés entre les deux panneaux de la paroi à double panneaux permettant aux parois d'être maintenues dressée par rapport au fond.
La coiffe comprend deux parois à double panneaux.
La direction des alvéoles de la coiffe est perpendiculaire à celle des alvéoles de la barquette.

### Présentation des figures

On décrit ci-après plus en détail un mode de réalisation de l'invention, non limitatif, en référence aux dessins annexés sur lesquels
- la figure 1 représente schématiquement la coupe d'une plaque alvéolaire en matière plastique alvéolaire ;
- la figure 2 montre une plaque en matériau plastique alvéolaire découpée et rainurée qui après mise en volume forme une barquette ;
- la figure 3 montre une une plaque en matériau plastique alvéolaire découpée et rainurée qui après mise en volume forme une barquette ;
- La figure 4 montre l'emballage avec la barquette et sa coiffe.

### Présentation d'un mode de réalisation de l'invention.

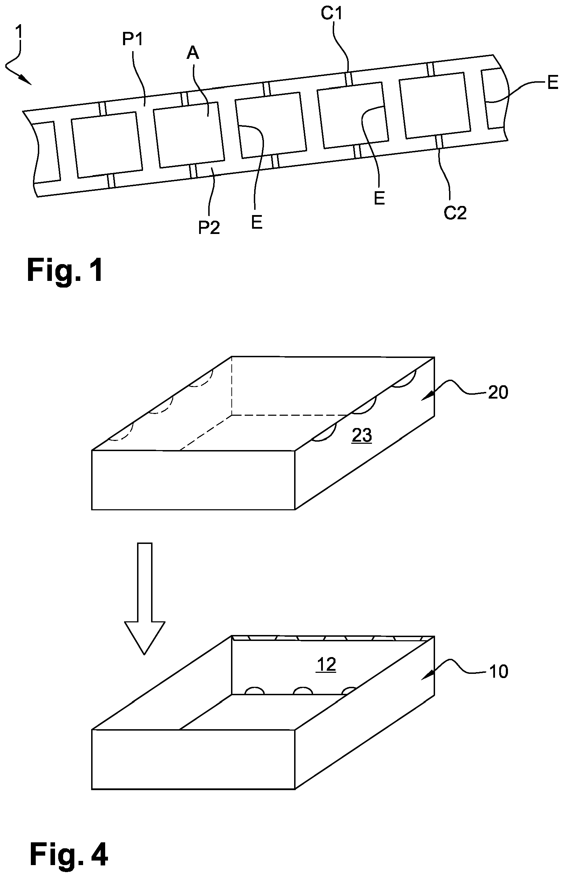

Sur la figure 1, une plaque 1 est représentée vue en coupe. Cette plaque présente deux feuilles couverture P1 et P2. Celles-ci sont reliées l'une à l'autre par des entretoises E parallèles entre elles. L'espace entre les feuilles de couverture et les entretoises forme des alvéoles A longitudinaux. Afin de rendre cette plaque perméable aux gaz et imperméable aux poussières on a percé au moins l'une des feuilles de couverture de trous de faible diamètre, celle formant la paroi intérieure de l'emballage. Ce diamètre est défini expérimentalement. Dans la pratique ces trous C1 ou C2 ont un diamètre compris entre 0,1 et 0,2 mm.

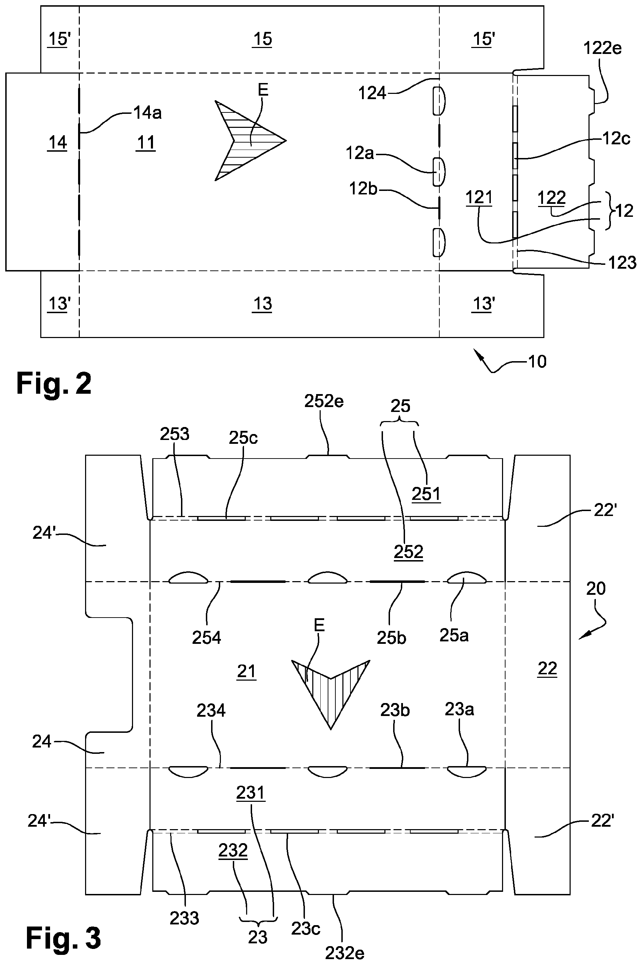

Sur la figure 2 est représentée une plaque découpée et rainée de manière à former la barquette de l'emballage. La face de la plaque à l'intérieur de la barquette est perforée de trous C. La face extérieure peut l'être aussi. De préférence, elle ne l'est pas. Les raines sont représentées en traits interrompus et les découpes en traits pleins. Cette plaque 10 comprend un panneau 11, formant le fond de la barquette, délimité par quatre lignes de pliage avec des panneaux latéraux formant les parois latérales : 12, 13, 14 et 15, de la barquette. Le panneau 12 perpendiculaire à la direction des entretoises E est double. Il est forme de deux panneaux : un premier panneau 121 articulé autour d'une ligne de pliage 124 du panneau de fond 11, un second panneau 122 articulé autour d'une ligne de pliage 123 autour du premier panneau 121. Des découpes 12c sont pratiquées le long de la ligne de pliage 123. Sur la figure 2 quatre sont représentées. Il s'agit de ne pas affaiblir la liaison entre les panneaux tout en ouvrant le plus d'alvéoles possibles. Des ouvertures sont aussi pratiquées le long de la ligne de pliage 124. Ces ouvertures 12a et 12b sont disposées en quinconce par rapport aux découpes 12c de telle façon que toutes les alvéoles communiquent avec l'atmosphère au moins par une extrémité. Une partie 12a des découpes est élargie pour former une entrée / sortie pour les gaz dans l'enceinte de l'emballage.

Des ergots 122 e sont ménagés le long du bord libre du panneau 122, en correspondance avec les ouvertures 12a. Le but est de verrouiller le panneau 122 en position contre le panneau 121 à la mise en volume par introduction des ergots dans les ouvertures 12a.

Sur ce mode de réalisation le panneau 14 opposé au panneau 12 est simple. Les alvéoles sont toutes ouvertes, néanmoins des découpes 14a sont prévues le long de la ligne de pliage pour la mise à l'air des alvéoles.

La figure 3 représente une plaque 20 découpée et rainée de manière à former la coiffe de l'emballage. La face de la plaque qui forme l'intérieur de la coiffe est perforée de trous C. La face extérieure peut l'être aussi. Les raines sont représentées en traits interrompus et les découpes en traits pleins.

Le panneau 21 formant le fond de la coiffe est délimité par des lignes de pliage avec des panneaux formant les parois latérales de la coiffe : 22, 23, 24, et 25. Les panneaux 23 et 25 dans la direction des entretoises E de la plaque sont doubles. 231, 232 et 251, 252 respectivement. Articulés autour de lignes de pliage, 234 et 254 respectivement avec le panneau de fond et 233 et 253 respectivement entre eux. Des découpes sont pratiquées le long des lignes de pliage 233 et 253, soit les découpes 23c et 25c respectivement. Comme les découpes 12c, leur nombre et leur longueur sont un compromis entre la tenue nécessaire entre les panneaux 231, 232 ; 251, 252 et la mise à l'atmosphère des alvéoles.

De la même façon, des découpes 23b resp 25b et ouvertures 23a resp 25a sont pratiquées le long des lignes de pliage, 234 et 254 respectivement, en quinconce avec les découpes 23c resp. 25c. Les ouvertures 23a et 25a ont les mêmes fonctions que les ouvertures 12a de la barquette.

Les panneaux 22 et 24 sont prolongés par des volets 22' et 24' agencés de façon à pouvoir être logés entre les panneaux des parois 23 et 25 à la mise en volume.

Pour la mise en volume de la barquette et de la coiffe, les panneaux latéraux sont dressés à angle droit par rapport au panneau de fond respectif et les volets sont placés contre les premiers panneaux 121, 231 et 251 des parois doubles. Les seconds panneaux sont ensuite rabattus sur les volets et les ergots 122e, 232e et 252e engagés dans les ouvertures correspondantes.

Dans ce mode de réalisation la paroi 14 est simple pour permettre le chargement de la barquette par ce coté, le panneau étant à plat.

Une fois les produits placés dans la barquette, on pose la coiffe et le tout est prêt pour la stérilisation. Le gaz pénètre en priorité par les ouvertures 12a, 23a et 25a. Inversement après stérilisation les échanges gazeux se font principalement par les ouvertures mais les découpes permettent un échange gazeux rapide aussi dans les alvéoles de la plaque grâce aux découpes.

La direction des alvéoles est perpendiculaire aux lignes d'articulation des panneaux double. Elle pourrait former un autre angle que droit mais elle n'est pas parallèle à celles-ci.

Les découpes peuvent être de simples entailles pratiquées avec une lame à travers une ou les deux feuilles de couverture. Elles peuvent aussi être pratiquées de manière à ce que les bords découpés soient espacés l'un de l'autre. L'espacement peut être de quelques millimètres.

Selon un autre mode de réalisation non représenté, on découpe des lamelles dans la feuille externe des panneaux de la caisse. Ces lamelles ont une faible largeur et s'étendent en travers de plusieurs alvéoles.

## Revendications

1. Emballage pour produits stériles comprenant une barquette (10) et une coiffe (20) en matériau plastique alvéolaire comportant au moins deux feuilles (P1, P2) de couverture maintenues à distance l'une de l'autre par une pluralité d'entretoises (E) ménageant des alvéoles longitudinales parallèles entre elles, la barquette et la coiffe comprenant un fond et des parois latérales découpés dans une plaque dudit matériau et dont au moins une desdites parois latérales (12 ; 23, 25) est à double épaisseur et formée par pliage de deux panneaux (121, 122 ; 231, 232 ; 251, 252) de la plaque autour de lignes de pliage perpendiculaires à la direction desdites entretoises, emballage **caractérisé par le fait que**
Les feuilles de couverture intérieures sont perforées pour permettre le passage d'un gaz de stérilisation au travers de ladite feuille de couverture, les perforations (C) permettant le passage du gaz mais empêchant le passage des poussières,
Des découpes (12a, 12b, 12c ; 23a, 23b, 23g ; 25a, 25b, 25c) larges traversent au moins l'autre feuille de couverture de la plaque pour assurer la communication desdites alvéoles avec l'extérieur et sont ménagées le long des lignes de pliage (123, 124 ; 233, 234 ; 253, 254) desdits panneaux.

2. Emballage selon la revendication précédente dont les perforations (C) sont ménagées sur la feuille de la plaque à l'intérieur de la caisse, et les découpes sur la feuille (P2) extérieure.

3. Emballage selon l'une des revendications précédentes dont des ouvertures (12a ; 23a ; 25a) sont pratiquées le long de la ligne de pliage (124 ; 234 ; 254) entre la paroi double (12 ; 23 ; 25) et le fond, ces ouvertures ayant pour fonction de recevoir des ergots (122e; 252e ; 232e) de verrouillage sur le bord libre du panneau extérieur et étant agencées pour permettre la circulation de gaz dans l'emballage depuis l'extérieur.

4. Emballage selon la revendication précédente dont des découpes sont pratiquées le long de ladite ligne de pliage de fond, les découpes et les ouvertures étant disposées en quinconce par rapport aux découpes sur la ligne de pliage des panneaux entre eux.

5. Emballage selon la revendication 4 dont une partie des alvéoles (A) débouche sur des découpes à leurs deux extrémités.

6. Emballage selon l'une des revendications précédentes dont la barquette (10) comprend une seule paroi latérale à double panneaux

7. Emballage selon l'une des revendications précédentes dont les parois adjacentes à ladite paroi latérale double comprennent des volets disposés entre les deux panneaux de la paroi à double panneaux permettant aux parois d'être maintenues dressée par rapport au fond.

8. Emballage selon l'une des revendications précédentes dont la coiffe (20) comprend deux parois à double panneaux.

9. Emballage selon la revendication précédente dont la direction des alvéoles de la coiffe est perpendiculaire à celle des alvéoles de la barquette.

## Patentansprüche

1. Verpackung für sterile Produkte, umfassend eine Schale (10) und einen Deckel (20) aus wabenförmigem Kunststoffmaterial, mindestens zwei Abdeckfolien (P1, P2) aufweisend, die durch eine Vielzahl von Abstandhaltern (E) voneinander beabstandet gehalten werden, die zueinander parallel angeordnete, längliche Wabenzellen ausbilden, wobei die Schale und der Deckel einen Boden und Seitenwände umfassen, die in einer Platte des Materials ausgeschnitten sind und von denen mindestens eine der Seitenwände (12; 23, 25) eine doppelte Stärke besitzt und durch Umbiegen von zwei Tafeln (121, 122; 231, 232; 251, 252) der Platte um Biegelinien gebildet wird, die senkrecht zur Richtung der Abstandhalter verlaufen, wobei die Verpackung **dadurch gekennzeichnet ist, dass**
die inneren Abdeckfolien perforiert sind, um den Durchlass eines Sterilisationsgases durch die Abdeckfolie zu ermöglichen, wobei die Perforationen (C) den Durchlass des Gases ermöglichen, aber den Durchlass von Staub verhindern,
breite Ausschnitte (12a, 12b, 12c; 23a, 23b, 23g; 25a, 25b, 25c) mindestens die andere Abdeckfolie der Platte durchsetzen, um die Kommunikation der Wabenzellen mit dem Außenbereich sicherzustellen, und entlang der Biegelinien (123, 124; 233, 234; 253, 254) der Tafeln ausgebildet sind.

2. Verpackung nach dem vorstehenden Anspruch, wobei die Perforationen (C) auf der Folie der Platte innerhalb der Kiste ausgebildet sind und die Ausschnitte auf der äußeren Folie (P2).

3. Verpackung nach einem der vorstehenden Ansprüche, wobei Öffnungen (12a; 23a; 25a) entlang der Biegelinie (124; 234; 254) zwischen der doppelten Wand (12; 23; 25) und dem Boden ausgeführt sind, wobei diese Öffnungen die Funktion haben, Vorsprünge (122e; 252e; 232e) zum Verriegeln an dem freien Ende der äußeren Tafel aufzunehmen, und angeordnet sind, um die Gaszirkulation in der Verpackung von außen zu ermöglichen.

4. Verpackung nach dem vorstehenden Anspruch, wobei Ausschnitte entlang der Biegelinie des Bodens ausgeführt sind, wobei die Ausschnitte und die Öffnungen in Bezug auf die Ausschnitte auf der Biegelinie der Tafeln zueinander in Quincunx-Anordnung bereitgestellt sind.

5. Verpackung nach Anspruch 4, wobei ein Teil der Wabenzellen (A) in Ausschnitte an ihren zwei Enden mündet.

6. Verpackung nach einem der vorstehenden Ansprüche, wobei die Schale (10) eine einzige Seitenwand mit doppelter Tafel umfasst.

7. Verpackung nach einem der vorstehenden Ansprüche, wobei die Wände, die an die doppelte Seitenwand angrenzen, Flügel umfassen, die zwischen den zwei Tafeln der Wand mit doppelter Tafel bereitgestellt sind, wodurch den Wänden ermöglicht wird, in Bezug auf den Boden aufrecht gehalten zu werden.

8. Verpackung nach einem der vorstehenden Ansprüche, wobei der Deckel (20) zwei Wände mit doppelter Tafel umfasst.

9. Verpackung nach dem vorstehenden Anspruch, wobei die Richtung der Wabenzellen des Deckels senkrecht zu jener der Wabenzellen der Schale verläuft.

## Claims

1. Packaging for sterile products, comprising a tray (10) and a cover (20) made of cellular plastics material comprising at least two cover sheets (P1, P2) separated from each other by a plurality of spacers (E) providing longitudinal cells that are parallel to each other, the tray and the cover comprising a base and sidewalls cut from a plate of said material and at least one of said sidewalls (12; 23, 25) being double-layered and formed by folding two panels (121, 122; 231, 232; 251, 252) of the plate around fold lines that are perpendicular to the direction of said spacers, which packaging is **characterised in that**
the internal cover sheets are perforated to allow a sterilisation gas to pass through said cover sheet, the perforations (C) allowing the passage of the gas but preventing the passage of dust,
wide cut-outs (12a, 12b, 12c; 23a, 23b, 23c; 25a, 25b, 25c) pass through at least the other cover sheet of the plate to ensure that said cells communicate with the exterior, and are provided along the fold lines (123, 124; 233, 234; 253, 254) of said panels.

2. Packaging according to the preceding claim, wherein the perforations (C) are made on the sheet of the plate inside the box and the cut-outs are made on the external sheet (P2).

3. Packaging according to any of the preceding claims, wherein openings (12a; 23a; 25a) are made along the fold line (124; 234; 254) between the double sidewall (12; 23; 25) and the base, said openings being designed to receive locking tabs (122e; 252e; 232e) on the free edge of the external panel and being arranged to allow gas to circulate inside the packaging from the exterior.

4. Packaging according to the preceding claim, wherein cut-outs are made along said base fold line, the cut-outs and the openings being arranged in a staggered manner relative to the cut-outs on the line for folding the panels together.

5. Packaging according to claim 4, wherein some of the cells (A) open onto cut-outs at both ends.

6. Packaging according to any of the preceding claims, wherein the tray (10) comprises a single double-panelled sidewall.

7. Packaging according to any of the preceding claims, wherein the sidewalls adjacent to said double sidewall comprise flaps arranged between the two panels of the double-panelled sidewall for holding the sidewalls straight relative to the base.

8. Packaging according to any of the preceding claims, wherein the cover (20) comprises two double-panelled sidewalls.

9. Packaging according to the preceding claim, wherein the direction of the cells of the cover is perpendicular to that of the cells of the tray.
